# EUROPEAN PATENT SPECIFICATION

(11) **EP 2 134 388 B1**
(45) Date of publication and mention of the grant of the patent: **19.12.2012**
(21) Application number: 08717258.1
(22) Date of filing: 29.02.2008
(51) Int. Cl.: A61M 5/142, A61M 5/145, A61M 5/148, A61M 5/14

(54) **A MEDICATION DELIVERY DEVICE COMPRISING A PLURALITY OF RESERVOIRS**
ARZNEIABGABEVORRICHTUNG MIT EINER VIELZAHL VON RESERVOIRS
DISPOSITIF D'ADMINISTRATION DE MÉDICAMENT COMPRENANT UNE PLURALITÉ DE RÉSERVOIRS

(30) Priority: 07.03.2007 EP 07103685
(43) Date of publication of application: 23.12.2009
(73) Proprietor: Novo Nordisk A/S, 2880 Bagsværd (DK)
(72) Inventor: GLEJBØL, Kristian, DK-2600 Glostrup (DK); MERSEBACH, Frank Daniel, DK-2900 Hellerup (DK); LANGKJÆR, Mette Ingrid, DK-2950 Vedbæk (DK); JEPPESEN, Per, DK-2700 Brønshøj (DK); MADSEN, Niels Berg, DK-4040 Jyllinge (DK)
(86) International application number: PCT/EP2008/052477
(87) International publication number: WO 2008/107378

(56) References cited:
- FR-A- 2 850 027
- US-A1- 2003 014 014
- US-A1- 2004 044 332
- US-A1- 2005 214 129

## Description

### FIELD OF THE I NVENTI ON

The present invention relates to a medication delivery device for delivering a liquid medicament from a plurality of reservoirs to the human body. The medication delivery device employs a displacement pump comprising two pump chambers, at least one of the chambers having variable displacement stroke volumes.

### BACKGROUND OF THE INVENTION

A prior art medication delivery device is disclosed in US 2002/007154. In the medication delivery device according to US 2002/007154 a liquid medicament is stored in a glass cartridge closed in the one end with a piston. Typically 3 ml. of a liquid medicament is stored in such glass cartridge. Furthermore, the medication delivery device of US 2002/007154 comprises a piston rod, acting on the piston, having a length sufficient to press the entire content of the glass cartridge out through a conduit mounted on the distal end of the medication delivery device. As disclosed in US 2002/007154 the piston rod is bendable in order to shorten the over all length of the medication delivery device, this bending however adds to the width of the delivery device.

Although one of the most refined medication delivery devices known in the art is the delivery device disclosed in US 2002/007154 it has nevertheless a number of important drawbacks.

A major drawback of a conventional medication delivery device of a type similar to the one disclosed in US 2002/007154 is that the only viable way for precise dosing of medicament is by controlled mechanical displacement of the piston. The displacement has to be extremely well controlled due to the large area of the dosing piston as even minute deviations from ideal piston position may result in either overdosing or under dosing of medicament. Another drawback of conventional medication delivery devices is that the length of the piston rod for expelling medicament from a cartridge needs to at least match the length of the cartridge containing the medicament. Thus, the length of the piston rod essentially dictates the overall length of the medication delivery device.

Another complicating problem with the above-mentioned type of medication delivery device is that comparably high forces are needed to displace the piston. Thus, high mechanical demands are put on the mechanical actuation system.

As a result of the need of a long piston rod and the high mechanical demands put on the mechanical actuation system delivery devices similar to the one described in US 2002/007154 tend to be rather bulky.

Smaller and more handy dosing systems are disclosed in the literature. A good example on a compact and portable system is given in WO 03/099358. Although small, the device disclosed in WO 03/099358 is a pre-filled single use auto injector. One important limitation of the system disclosed in WO 03/099358 is that it lacks the dose setting flexibility often required in modern therapy. If e.g. the medicament is needed for treatment of diabetes mellitus a wide range of doses is needed. The exact dose to be delivered to a patient depends among other things on the recent carbohydrate intake and on the amount of recent exercise. Thus, efficient treating of a disease like diabetes mellitus requires that the device can deliver a range of doses.

A great number of different dosing systems are described in the literature. Among these many of the described systems are small and convenient to use while others are precise and offers the flexibility required for treatment of complex diseases like diabetes mellitus. At the time of writing there is, however, a need for small, precise dosing systems that are at the same time simple and convenient to use.

Furthermore, some modern therapies require the administration of multiple non-miscible substances. In the literature only sparse information is disclosed on devices suitable for delivering more than one drug in a single injection.

It may be seen as an object of the present invention to provide a novel strategy for dosing systems having the virtues of advanced devices as described in 2002/007154 but the size and convenience of the simple devices as exemplified by WO 03/099358.

Document FR-A-2 850 027 describes a medication delivery device according to the preamble of claim 1.

It may be seen as a further object of the present invention to provide a medication delivery device from which set doses of medicament can be administered from a plurality of medicament containing reservoirs, said device being smaller and lighter compared to the present state of the art.

### SUMMARY OF THE I NVENTI ON

The above-mentioned objects and other objects are complied with by substituting the glass cartridge normally employed in flexible dosing systems with a collapsible reservoir special in that the pressure difference between the inside of the reservoir and the ambient is very small. This reservoir may be combined with a displacement pump comprising a plurality of pump chambers where the displacement stroke volumes of each pump camber can be adjusted in accordance with specific needs.

A very precise and light dosing system can be obtained based on a collapsible reservoir and a displacement pump having adjustable displacement volumes. The most important virtue of a collapsible reservoir is in this context its pressure neutrality, i.e. the pressure inside the reservoir is approximately the same as the pressure outside the reservoir. Other important virtues over normal non-collapsible glass cartridge based reservoirs are low weight, compactness and low manufacturing costs. By taking advantage of the pressure neutrality it is possible to employ a displacement pump having adjustable displacement volumes to deliver very precise amounts of medicament.

If a displacement pump is to be applied on a standard glass cartridge in a reliable manner, a force must be applied to the piston in order to overcome the erratic friction between the piston and the glass vessel. This would imply a permanently pressurised cartridge as the friction between the piston and the glass vessel is highly variable. However, a pressurised cartridge is not acceptable as failure of the pump may result in overdosing of medicament.

By avoiding the traditional piston rod and drive mechanism great simplification can be gained in the design of the medication delivery device.

If the medication delivery device is to be operated by electromechanical means the number of strokes required to deliver a given dose can be chosen randomly at no cost in the complexity of the system.

If the medication delivery device is to be operated manually or by a simple spring actuated mechanisms, the operation of the device is highly simplified if the complete dose of medicament is measured and delivered in a single stroke cycle. If the mechanical design of the device is such that only a single stroke of medicament can be delivered this furthermore improves the safety of the device significantly since multiple doses can not be delivered due to mechanical or electrical malfunctions.

By making a part of the collapsible reservoir from for example a sheet-like material it is possible to make a reservoir that is easily collapsible if the pressure outside the reservoir exceeds the inner pressure of the reservoir. As explained in details later, the term "collapsible" is not limited to reservoirs where the outer surface can collapse. This definition does also apply to a reservoir comprising a rigid outer shell but having an inner collapsible membrane made from a sheet-like material.

If a very simple, cheap and robust medication delivery device is wanted direct actuation of the pumping means may be the best choice. Direct actuation would typically be the preferred option for a third world device or a device containing a critical lifesaving drug. Direct actuation of the pumping unit may furthermore be an option if the mechanical actuation of the pumping unit fails.

Thus, in a first aspect the present invention relates to a medication delivery device for delivering a set dose of medicament, the medication delivery device comprising
- pump means comprising first and second pump chambers,
- a first collapsible reservoir adapted to contain medicament, the first pump chamber being adapted to transfer medicament from the first collapsible reservoir to an outlet arrangement, said outlet arrangement being in fluidic communication with an associated hypodermic needle,
- a second reservoir adapted to contain a liquid substance, the second pump chamber being adapted to transfer substance from the second reservoir to the outlet arrangement,
wherein the first pump chamber is adapted to deliver the set dose of medicament during one or more pump strokes, the stroke volume(s) of said one or more pump strokes being variable.

Similarly, the second pump chamber may be adapted to deliver substance during one or more pump strokes.

In the present content the term "collapsible" should be interpreted broadly. Thus, collapsible is to cover a reservoir comprising a flexible sheet-like material which changes its form with changes of the volume of the reservoir. In addition, the term collapsible is also to cover any arrangement which allows changes in a volume of a reservoir. Such changes in volume could be provided by moveable wall portions of the reservoir as long as the pressure inside the reservoir maintains at approximately the same level as the pressure outside the reservoir.

In the context of the present invention, "hypodermic needle" should be interpreted broadly, i.e. comprising injection needles, infusion sets, micro-needle arrays or other suitable means for mechanically penetrating the dermis, hereby allowing for infusion of a substance.

A pressure difference of around 0.1 bar between the interior of the first collapsible reservoir and the surroundings may be acceptable. However, it is an advantage of the present invention that the interior pressure in the first collapsible reservoir is kept at essentially the same level - independent of the amount of medicament in the first collapsible reservoir.

The medication contained in the first collapsible reservoir may in principle be any kind of medication, such as one or more peptides, one or more proteins or a combination hereof. Thus, the peptides or proteins may comprise insulin, insulin analogues, GLP or GLP analogues or a mixture comprising one or more of these.

The pump means and the first collapsible reservoir may be rigidly arranged relative to each other. Such a rigidly arrangement between reservoir and pump means may be established by attaching at least part of the reservoir directly to a part of the pump means. The pump means and the collapsible reservoir may be arranged within an at least partly closed shell or housing. Openings allowing setting of a dose to be expelled may be provided.

The first collapsible reservoir may comprise a substantially rigid portion and a collapsible portion, the collapsible portion being adapted to collapse into at least part of the substantially rigid portion upon changing the volume of the first collapsible reservoir. Part of an inner surface of the collapsible portion of the first collapsible reservoir may comprise a sheet material. The second reservoir may be a second collapsible reservoir also comprising a substantially rigid portion and a collapsible portion, the collapsible portion being adapted to collapse into at least part of the substantially rigid portion upon changing the volume of the second collapsible reservoir. Similar to the first collapsible reservoir, part of an inner surface of the collapsible portion of the second collapsible reservoir may comprise a sheet material.

The sheet-like material may comprise a sheet comprising a thermoplastic material which may form part of a multilayer sheet structure. The sheet material may further comprise one or more barrier layers. The sheet material may have a thickness smaller than 1 mm, such as smaller than 0.8 mm, such as smaller than 0.5 mm, such as smaller than 0.3 mm.

The medicament contained in the first collapsible reservoir may be sucked out of the first reservoir applying displacement pump means in the first pump chamber. Similarly, the medicament contained in a second collapsible reservoir may be sucked out of the second reservoir applying displacement pump means in the second pump chamber.

A displacement stroke and/or a restoring stroke of a pump cycle may at least partly be actuated by the user of the medication delivery device. Thus, the appliance of a force by the user of the medication delivery device may at least partly be utilized to expel the medicament from the first and/or second pump chamber. Alternatively, or in addition, the displacement stroke and/or a restoring stroke of a pump cycle may at least partly be actuated by an energising mechanism. This energising mechanism may comprise a spring, such as a torsion spring or a linear spring, an elastomeric element, a volume of compressed air, or a combination thereof. Finally, the displacement stroke and/or a restoring stroke of a pump cycle may at least partly be actuated by an electromechanical actuator being controlled by an electronic control circuit comprising a microprocessor.

The first and second pump chambers may be arranged to deliver medicament and substance to the outlet arrangement essentially simultaneously. In this configuration medicament and substance may be mixed in the outlet arrangement and/or in the associated hypodermic needle. It should be noted that medicament and substance are preferably kept separated as long as they are in the first and second pump chambers, respective. Alternatively, the first and second pump chambers are arranged to deliver medicament and substance to the outlet arrangement in a sequential manner. The order of delivery may be chosen arbitrarily. To comply with demands for delivering medicament and substance in a sequential manner the medication delivery may further comprise delivery delay means, said delivery delay means being adapted to delay delivery of substance from the second pump chamber. The delivery delay means may comprise a resilient member, such as a spring, said resilient member being operatively connected to a displaceable piston acting on substance in the second pump chamber. Alternatively, the delivery delay means may comprise a tube section with reduced flow dimensions, said tube section being in fluidic communication with the second pump chamber and the outlet arrangement.

The medication delivery device according to the present invention may further comprise dose counting means which allows the user of the medication delivery device to set the dose of medicament to be expelled. The medication delivery device may further comprise end of content indicating means which informs the user of the medication delivery device that the collapsible reservoir is empty, or close to being empty, and, thus, needs to be replaced.

The medication delivery device may further comprise means for assisting the user of the medication delivery device deciding on the proper dose of medication. This assisting means may at least partly form part of a module, said module being adapted to be secured to the medication delivery device. A control unit may further be provided. The control unit may be adapted to communicate with the medication delivery device and/or with the module secured thereto.

The medication delivery device may further comprise at least one display member, said display member being arranged on the medication delivery device, on the module being adapted to be secured to the medication delivery device or as part of the control unit adapted to communicate with for example the medication delivery device.

The medication delivery device, the module adapted to be secured to the medication delivery device or the control unit may further comprise at least one microcontroller arranged in the medication delivery device, the attached module or in the control unit. The microcontroller may facilitate that dose information is fed to electromechanical means for controlling the delivered dose.

In order to power the medication delivery device according to the present invention the device may comprise power supplying means, such as a battery. Finally, the medication delivery device may be equipped with a hypodermic needle.

In a second aspect, the present invention relates to a method for delivering a set dose of medicament from a medication delivery device, the method comprising the steps of
- providing pump means comprising first and second pump chambers,
- providing a first collapsible reservoir containing medicament and transferring medicament from the first collapsible reservoir to an outlet arrangement, said outlet arrangement being in fluidic communication with an associated hypodermic needle,
- providing a second reservoir containing a liquid substance and transferring substance from the second reservoir to the outlet arrangement,
delivering the transferred medicament and liquid substance during one or more pump strokes, the stroke volume(s) of said one or more pump strokes being variable.

Thus, according to the present invention the stroke volume is set in accordance with the dose of medicament to be expelled. This implies that the stroke volume may be set to expel the set dose in a single pump stroke, or in a series of pump strokes with potentially different stroke volumes. Thus, a maximum of ten, eight, six, four or two pump strokes may be applied to expel the set dose of medicament. As already mentioned a single pump stroke may also be applied to expel the complete dose.

Similarly, a set dose of medicament may be expelled using a first pump stroke having a first stroke volume, said first pump stroke being followed by a second pump stroke having a second stroke volume, wherein the first stroke volume is different from the second stroke volume. The first and second stroke volumes may also be equal, as well as the number of applied pump strokes may differ from two.

### BRI EF DESCRIPTION OF THE DRAWINGS

The present invention will now be explained in further details with reference to the accompanying figures, wherein
Fig. 1 shows an example of a possible layout of the main working parts of mechanics of a medication delivery device according to the present invention,
Fig. 2 shows how medicament and substance are pumped into first and second pump chambers,
Fig. 3 shows how medicament and substance are expelled from the first and second pump chambers, and
Fig. 4 shows a medication delivery device capable of delivering medicament and substance in a sequential manner.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the spirit and scope of the invention as defined by the appended claims.

### DETAILED DESCRI PTI ON OF THE INVENTION

In its most general aspect the present invention relates to a medication delivery device comprising some sort of displacement pump having adjustable pumping stroke volumes and a plurality of medicament containing reservoirs being in fluidic communication with respective pump chambers. This arrangement facilitates that the medication delivery device is capable of expelling an arbitrary preset dose of medication by applying one or more adjusted pump strokes. As the delivered doses not only depend on the number of pumping strokes but also on the chosen stroke volumes very high dose precisions can be obtained using one or few pumping strokes.

Thus, it is an advantage of the medication delivery device according to the present invention that the delivered dose is not proportional to the number of pumping strokes as the stroke volumes of the pump arrangement can be adjusted to match a set dose of medication to be expelled.

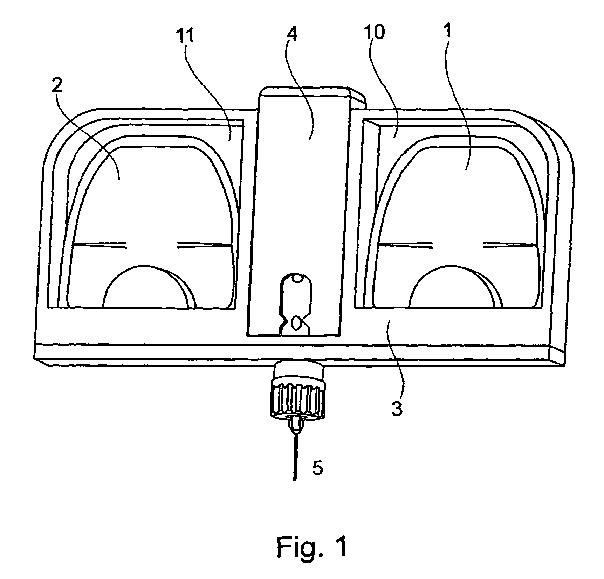

The functioning of an exemplary medication delivery device according to the present invention will now be explained with reference to Figs. 1, 2 and 3. Central to the medication delivery device is at least one collapsible reservoir 1. The additional second reservoir 2 may optionally be a collapsible reservoir. The collapsible reservoir 1 can be made in a number of different ways all having in common that at least a part of the inner side of the reservoir is collapsible and that there is only a minor pressure difference between the inside of the reservoir and the surroundings. By collapsible is thus meant that the reservoir is capable of changing its volume by having a collapsible or flexible inner wall structure whereby the volume of the reservoir is changeable while keeping a minor pressure difference between the inside of the reservoir and the surroundings.

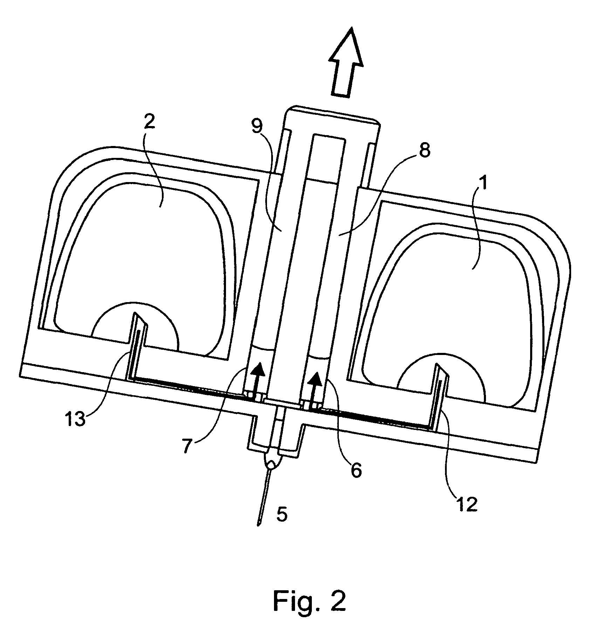

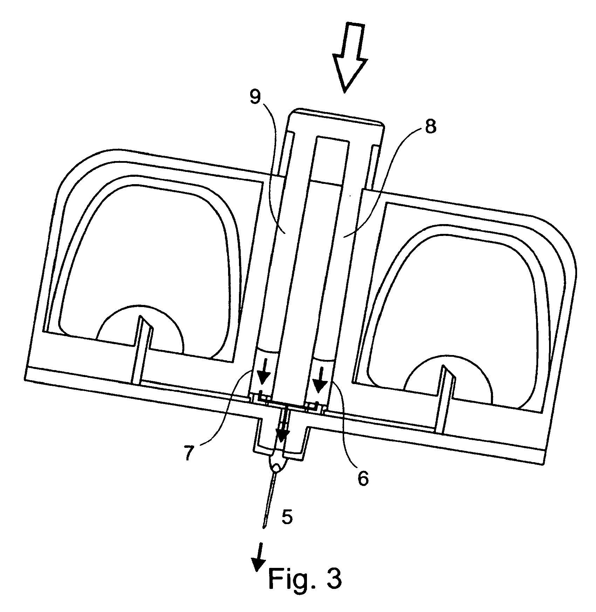

In Fig. 1 an embodiment of a medication delivery device is depicted in a cross-sectional view. As seen in Fig. 1 the two reservoirs 1,2 are attached to a housing structure 3 to which the displacement pump 4 and an injection needle 5 are also attached. The reservoirs 1,2 are positioned in respective hollow portions 10, 11 of the housing structure 3. For clarity reasons the detailed functioning of the pumping mechanism is shown in Figs. 2 and 3.

Referring now to Fig. 2, the overall functioning of the medication delivery device is that medication is drawn from reservoirs 1,2 to respective pumping chambers 6,7 via conduits 12,13 by retraction of the pistons 8,9 as indicated by the arrow. As the retraction of the pistons can be adjusted the volumes of medication measured to pump chambers 6,7 are adjustable. The medicaments of the two reservoirs are kept completely separated in pumping chambers 6,7. In this way unintentional chemical reactions in the pumping chambers are avoided. Each conduit 12,13 comprises a one-way valve so that medication is prevented from returning to the reservoirs when medications present in pump chambers 6,7 are pressurised by pistons 8,9.

To deliver the medication measured to the pump chambers 6,7, pistons 8,9 are repositioned to their original positions - see Fig. 3. Upon repositioning of the pistons 8,9 the respective pump chambers are emptied and the medication of pump chamber 8 is mixed with the medication of pump chamber 7 as both medications are expelled from the medication delivery device via the injection needle 5.

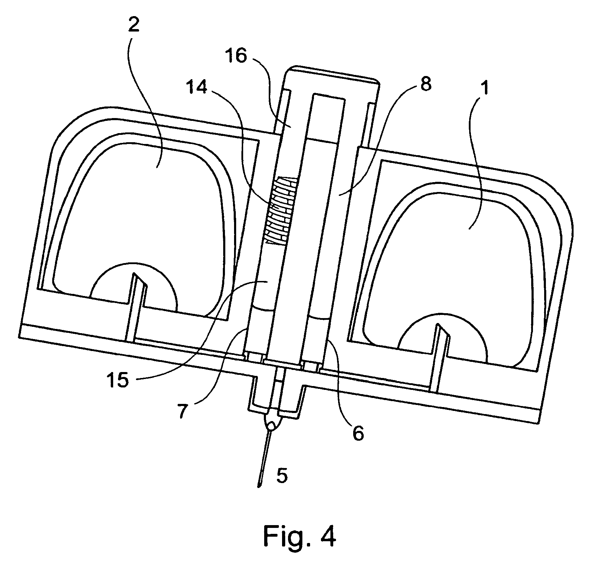

Fig. 4 shows a medication delivery device capable of delivering medications from the two reservoirs in a sequential manner. In order to achieve this, a mechanical delay arrangement 14,15 is depicted in Fig. 4. The mechanical delay arrangement functions in the following way: Pumps chambers 6,7 are filled with medication as shown in Fig. 2. When pistons 8 and 16 are repositioned in order to expel medications from the device linear spring 14 is compressed leaving piston 15 in an essentially unchanged position. While the position of piston 15 is essentially unchanged piston 8 is allowed to return to its original position whereby the medication of pump chamber 6 is expelled via the injection needle 5. When pump chamber 6 is empty the length of linear spring extends to its original length whereby piston 15 is moved forward causing pump chamber 7 to be emptied. In this way medications of pump chambers 6,7 are delivered in an sequential manner.

In its simplest form a collapsible reservoir to be used in a medication delivery device according to the present invention is made from sheet material which is folded and welded, thus forming a closed bag. If this type of collapsible reservoir is employed it is normally necessary to attach some sort of coupling unit to the reservoir. Although the sheet material for a simple reservoir can be chosen from a wide range of materials, the preferred materials are thermoplastics or laminates containing at least one layer of thermoplastic material. The sheet material should fulfil a number of different demands if employed for production of a reservoir. Most important is that the reservoir should have excellent barrier properties and be compatible with the medication to be stored in the reservoir. Additionally, the material should be processable, i.e. if welding is chosen as the preferred process of joining the sheet material the material should be weldable. Additionally, the material should be able to withstand the mechanical loads to which it will be subjected during processing, transport and use. A final demand often put on the sheet material is that it should be possible to sterilize the material without critical degradation.

Due to the many conflicting demands on the sheet material, the sheet material may be a multilayer structure made from two or more layers having different properties. The sheet material will often be made predominantly from a laminate of multiple thermoplastic layers having the required mechanical properties. One or more barrier layers will be sandwiched between thermoplastic layers. Among inorganic barrier layers inorganic materials like AI AIOₓ, AIₓO_{y}N_{z}, SiOₓ, SiOₓN_{y}, SiNₓ are preferred. The numbers x,y,z does not refer to any specific stochiometric composition but rather indicate a range of numbers as barrier layers often are non-stochiometric substances. Among organic barrier layers polyvinylchloride (PVC), polyparylene, cyclo olefin copolymer (COC) polypropylene (PP) and polychlorotrifluoroethylene (PCTFE) are preferred materials. Among these PP, PVC, COC and PCTFE have a high mechanical strength. They may hence be used either in a laminate or as single layer sheets.

The sheet thickness strongly depends on the stiffness and barrier properties of the sheet material. In a preferred embodiment of the present invention the average thickness of the sheet material is less than 1 mm. In a more preferred embodiment of the invention the average thickness of the sheet material is less than 0.3 mm.

Depending on the properties of the sheet material a number of different strategies for joining may be employed, including adhesive bonding, welding and mechanical joining. Among these welding, preferably laser welding, RF welding or heat welding are preferred.

If a coupling unit is to be attached to the reservoir this coupling unit has to be made from a material which is compatible with the material of the reservoir. The coupling unit can either be a flexible rubber septum or a rigid coupling unit.

Evidently, the medication delivery device according to the present invention facilitates injection of in principle any fluid, solution or suspension containing any combination of therapeutic proteins and/or peptides. In a preferred embodiment the injected medication comprises insulin, insulin analogues, GLP or GLP analogues especially suitable for treatment of diabetes. In an equally preferred embodiment the injected medication comprises human growth hormones or human growth hormone analogues.

## Claims

1. A medication delivery device for delivering a set dose of medicament, the medication delivery device comprising
- pump means comprising first and second pump chambers,
- a first collapsible reservoir adapted to contain medicament, the first pump chamber being adapted to transfer medicament from the first collapsible reservoir to an outlet arrangement, said outlet arrangement being in fluidic communication with an associated hypodermic needle,
**characterized in that** it further comprises:
- a second reservoir adapted to contain a liquid substance, the second pump chamber being adapted to transfer substance from the second reservoir to the outlet arrangement,
wherein the first pump chamber is adapted to deliver the set dose of medicament during one or more pump strokes, the stroke volume(s) of said one or more pump strokes being variable.

2. A medication delivery device according to claim 1, wherein the second pump chamber is adapted to deliver substance during one or more pump strokes, the stroke volume(s) of said one or more pump strokes being variable.

3. A medication delivery device according to claim 1 or 2, wherein the first and second pump chambers are arranged to deliver medicament and substance to the outlet arrangement essentially simultaneously.

4. A medication delivery device according to claim 1 or 2, wherein the first and second pump chambers are arranged to deliver medicament and substance to the outlet arrangement in a sequentially manner.

5. A medication delivery device according to claim 4, further comprising delivery delay means, said delivery delay means being adapted to delay delivery of substance from the second pump chamber.

6. A medication delivery device according to claim 5, wherein the delivery delay means comprises a resilient member, such as a spring, said resilient member being operatively connected to a displaceable piston acting on substance in the second pump chamber.

7. A medication delivery device according to claim 5, wherein the delivery delay means comprises a tube section with reduced flow dimensions, said tube section being in fluidic communication with the second pump chamber and the outlet arrangement.

8. A medication delivery device according to any of the preceding claims, wherein the first collapsible reservoir comprises a substantially rigid portion and a collapsible portion, the collapsible portion being adapted to collapse into at least part of the substantially rigid portion upon changing the volume of the collapsible reservoir.

9. A medication delivery device according to claim 8, wherein the collapsible portion of the reservoir comprises a sheet material.

10. A medication delivery device according to claim 9, wherein the sheet material comprises a thermoplastic material.

11. A medication delivery device according to claim 10, wherein the thermoplastic material forms part of a multilayer sheet structure.

12. A medication delivery device according to any of claims 9-11, wherein the sheet material further comprises one or more barrier layers.

13. A medication delivery device according to any of claims 9-12, wherein the sheet material has a thickness smaller than 1 mm, such as smaller than 0.8 mm, such as smaller than 0.5 mm, such as smaller than 0.3 mm.

14. A medication delivery device according to any of the preceding claims, wherein the second reservoir comprises a second collapsible reservoir comprises a substantially rigid portion and a collapsible portion, the collapsible portion being adapted to collapse into at least part of the substantially rigid portion upon changing the volume of the collapsible reservoir.

15. A medication delivery device according to claim 14, wherein the collapsible portion of the reservoir comprises a sheet material, such as a sheet material comprising a thermoplastic material.

16. A medication delivery device according to claim 15, wherein the thermoplastic material forms part of a multilayer sheet structure.

17. A medication delivery device according to claim 15 or 16, wherein the sheet material further comprises one or more barrier layers.

18. A medication delivery device according to any of claims 15-17, wherein the sheet material has a thickness smaller than 1 mm, such as smaller than 0.8 mm, such as smaller than 0.5 mm, such as smaller than 0.3 mm.

19. A medication delivery device according to any of the preceding claims, wherein a displacement stroke and/or a restoring stroke of a pump cycle are/is at least partly actuated by the user of the medication delivery device.

20. A medication delivery device according to any of the preceding claims, wherein a displacement stroke and/or a restoring stroke of a pump cycle are/is at least partly actuated by a spring mechanism.

21. A medication delivery device according to any of the preceding claims, further comprising a hypodermic needle.

## Patentansprüche

1. Medikamentenabgabevorrichtung zum Abgeben einer eingestellten Medikamentendosis, wobei die Medikamentenabgabevorrichtung Folgendes umfasst:
- eine Pumpe, die eine erste und eine zweite Pumpenkammer umfasst,
- einen ersten zusammenklappbaren Behälter, der zum Aufnehmen eines Medikaments angepasst ist, wobei die erste Pumpenkammer derart angepasst ist, das Medikament von dem ersten zusammenklappbaren Behälter zu einer Auslassanordnung zu transferieren, wobei die Auslassanordnung mit einer damit verbundenen Injektionsnadel in Fluidkommunikation ist,
**dadurch gekennzeichnet, dass** sie des Weiteren umfasst:
- einen zweiten Behälter, der zum Aufnehmen einer flüssigen Substanz angepasst ist, wobei die zweite Pumpenkammer derart angepasst ist, die Substanz vom zweiten Behälter zu der Auslassanordnung zu transferieren,
wobei die erste Pumpenkammer derart angepasst ist, die eingestellte Medikamentendosis während einem oder mehreren Pumpenstößen abzugeben, und das Stoßvolumen bzw. die Stoßvolumina des einen oder der mehreren Pumpenstöße variabel ist.

2. Medikamentenabgabevorrichtung nach Anspruch 1, wobei die zweite Pumpenkammer derart angepasst ist, die Substanz während einem oder mehreren Pumpenstößen abzugeben, und das Stoßvolumen bzw. die Stoßvolumina des einen oder der mehreren Pumpenstöße variabel ist.

3. Medikamentenabgabevorrichtung nach Anspruch 1 oder 2, wobei die erste und die zweite Pumpenkammer derart angeordnet sind, dass sie das Medikament und die Substanz im Wesentlichen gleichzeitig an die Auslassanordnung abgeben.

4. Medikamentenabgabevorrichtung nach Anspruch 1 oder 2, wobei die erste und die zweite Pumpenkammer derart angeordnet sind, dass sie das Medikament und die Substanz in aufeinanderfolgender Weise an die Auslassanordnung abgeben.

5. Medikamentenabgabevorrichtung nach Anspruch 4, des Weiteren umfassend ein Abgabeverzögerungsmittel, wobei das Abgabeverzögerungsmittel derart angepasst ist, die Abgabe der Substanz von der Pumpenkammer zu verzögern.

6. Medikamentenabgabevorrichtung nach Anspruch 5, wobei das Abgabeverzögerungsmittel ein elastisches Element wie eine Feder umfasst, wobei das elastische Element mit einem verschiebbaren Kolben, der auf die Substanz in der zweiten Pumpenkammer einwirkt, funktionsfähig verbunden ist.

7. Medikamentenabgabevorrichtung nach Anspruch 5, wobei das Abgabeverzögerungsmittel einen Rohrabschnitt mit reduzierten Flussmaßen umfasst, wobei der Rohrabschnitt mit der zweiten Pumpenkammer und der Auslassanordnung in Fluidkommunikation ist.

8. Medikamentenabgabevorrichtung nach einem der vorangehenden Ansprüche, wobei der erste zusammenklappbare Behälter einen im Wesentlichen starren Teil und einen zusammenklappbaren Teil umfasst, wobei der zusammenklappbare Teil derart angepasst ist, durch Ändern des Volumens des zusammenklappbaren Behälters zu zumindest einem Teil des im Wesentlichen starren Teils zusammenzuklappen.

9. Medikamentenabgabevorrichtung nach Anspruch 8, wobei der zusammenklappbare Teil des Behälters ein Lagenmaterial umfasst.

10. Medikamentenabgabevorrichtung nach Anspruch 9, wobei das Lagenmaterial ein thermoplastisches Material umfasst.

11. Medikamentenabgabevorrichtung nach Anspruch 10, wobei das thermoplastische Material einen Teil einer mehrschichtigen Lagenstruktur bildet.

12. Medikamentenabgabevorrichtung nach einem der Ansprüche 9-11, wobei das Lagenmaterial des Weiteren eine oder mehrere Sperrschichten umfasst.

13. Medikamentenabgabevorrichtung nach einem der Ansprüche 9-12, wobei das Lagenmaterial eine Dicke aufweist, die kleiner als 1 mm, wie kleiner als 0,8 mm, wie kleiner als 0,5 mm, wie kleiner als 0,3 mm ist.

14. Medikamentenabgabevorrichtung nach einem der vorangehenden Ansprüche, wobei der zweite Behälter einen zweiten zusammenklappbaren Behälter umfasst, der einen im Wesentlichen starren Teil und einen zusammenklappbaren Teil umfasst, wobei der zusammenklappbare Teil derart angepasst ist, durch Ändern des Volumens des zusammenklappbaren Behälters zu zumindest einem Teil des im Wesentlichen starren Teils zusammenzuklappen.

15. Medikamentenabgabevorrichtung nach Anspruch 14, wobei der zusammenklappbare Teil des Behälters ein Lagenmaterial wie ein Lagenmaterial, das ein thermoplastisches Material umfasst, umfasst.

16. Medikamentenabgabevorrichtung nach Anspruch 15, wobei das thermoplastische Material einen Teil einer mehrschichtigen Lagenstruktur bildet.

17. Medikamentenabgabevorrichtung nach Anspruch 15 oder 16, wobei das Lagenmaterial des Weiteren eine oder mehrere Sperrschichten umfasst.

18. Medikamentenabgabevorrichtung nach einem der Ansprüche 15-17, wobei das Lagenmaterial eine Dicke aufweist, die kleiner als 1 mm, wie kleiner als 0,8 mm, wie kleiner als 0,5 mm, wie kleiner als 0,3 mm ist.

19. Medikamentenabgabevorrichtung nach einem der vorangehenden Ansprüche, wobei ein Verschiebungsstoß und/oder ein zurückversetzender Stoß eines Pumpenzyklus zumindest teilweise durch den Anwender der Medikamentenabgabevorrichtung betätigt wird/werden.

20. Medikamentenabgabevorrichtung nach einem der vorangehenden Ansprüche, wobei ein Verschiebungsstoß und/oder ein zurückversetzender Stoß eines Pumpenzyklus zumindest teilweise durch einen Federmechanismus betätigt wird/werden.

21. Medikamentenabgabevorrichtung nach einem der vorangehenden Ansprüche, des Weiteren umfassend eine Injektionsnadel.

## Revendications

1. Un dispositif de délivrance d'une médication pour délivrer une dose ajustée de médicament, le dispositif de délivrance de médication comprenant :
- des moyens formant pompe comprenant une première et une seconde chambre de pompe,
- un premier réservoir écrasable apte à contenir un médicament, la première chambre de pompe étant apte à transférer le médicament du premier réservoir écrasable à une configuration d'orifice de sortie, ladite configuration d'orifice de sortie étant en communication de fluide avec une aiguille hypodermique associée,
**caractérisé en ce qu'**il comprend en outre :
- un second réservoir apte à contenir une substance liquide, la seconde chambre de pompe étant apte à transférer la substance du second réservoir à la configuration d'orifice de sortie,
dans lequel la première chambre de pompe est apte à délivrer la dose ajustée de médicament durant une ou plusieurs courses de la pompe, le ou les volumes de la course desdites une ou plusieurs courses de pompe étant variables.

2. Un dispositif de délivrance de médication selon la revendication 1, dans lequel la seconde chambre de pompe est apte à délivrer la substance durant une ou plusieurs courses de la pompe, le ou les volumes de la course desdites une ou plusieurs courses de pompe étant variables.

3. Un dispositif de délivrance de médication selon la revendication 1 ou 2, dans lequel les première et seconde chambres de pompe sont configurées pour délivrer le médicament et la substance à la configuration d'orifice de sortie de façon essentiellement simultanée.

4. Un dispositif de délivrance de médication selon la revendication 1 ou 2, dans lequel la première et la seconde chambre de pompe sont configurées pour délivrer le médicament et la substance à la configuration d'orifice de sortie d'une manière séquentielle.

5. Un dispositif de délivrance de médication selon la revendication 4, comprenant en outre des moyens de retard de délivrance, les moyens de retard de délivrance étant aptes à retarder la délivrance de la substance depuis la seconde chambre de pompe.

6. Un dispositif de délivrance de médication selon la revendication 5, dans lequel les moyens de retard de délivrance comprennent un organe élastique, tel qu'un ressort, ledit organe élastique étant couplé de manière opérante à un piston déplaçable agissant sur la substance dans la seconde chambre de pompe.

7. Un dispositif de délivrance de médication selon la revendication 5, dans lequel les moyens de retard de délivrance comprennent une section tubulaire avec des dimensions d'écoulement réduites, ladite section tubulaire étant en communication de fluide avec la seconde chambre de pompe et avec la configuration d'orifice de sortie.

8. Un dispositif de délivrance de médication selon l'une des revendications précédentes, dans lequel le premier réservoir écrasable comprend une partie substantiellement rigide et une partie écrasable, la partie écrasable étant apte à s'écraser dans au moins une partie de la partie substantiellement rigide aux changements de volume du réservoir écrasable.

9. Un dispositif de délivrance de médication selon la revendication 8, dans lequel la partie écrasable du réservoir comprend un matériau en feuille.

10. Un dispositif de délivrance de médication selon la revendication 9, dans lequel le matériau en feuille comprend un matériau thermoplastique.

11. Un dispositif de délivrance de médication selon la revendication 10, dans lequel le matériau thermoplastique forme une partie d'une structure en feuille multicouche.

12. Un dispositif de délivrance de médication selon l'une des revendications 9 à 11, dans lequel le matériau en feuille comprend en outre une ou plusieurs couches de barrière.

13. Un dispositif de délivrance de médication selon l'une des revendications 9 à 12, dans lequel le matériau en feuille présente une épaisseur inférieure à 1 mm, telle qu'inférieure à 0,8 mm, telle qu'inférieure à 0,5 mm, telle qu'inférieure à 0,3 mm.

14. Un dispositif de délivrance de médication selon l'une des revendications précédentes, dans lequel le second réservoir comprend un second réservoir écrasable qui comprend une partie substantiellement rigide et une partie écrasable, la partie écrasable étant apte à s'écraser dans au moins une partie de la partie substantiellement rigide aux changements du volume du réservoir écrasable.

15. Un dispositif de délivrance de médication selon la revendication 14, dans lequel la partie écrasable du réservoir comprend un matériau en feuille, tel qu'un matériau en feuille comprenant un matériau thermoplastique.

16. Un dispositif de délivrance de médication selon la revendication 15, dans lequel le matériau thermoplastique forme une partie d'une structure en feuille multicouche.

17. Un dispositif de délivrance de médication selon la revendication 15 ou 16, dans lequel le matériau en feuille comprend en outre une ou plusieurs couches de barrière.

18. Un dispositif de délivrance de médication selon l'une des revendications 15 à 17, dans lequel le matériau en feuille présente une épaisseur inférieure à 1 mm, telle qu'inférieure à 0,8 mm, telle qu'inférieure à 0,5 mm, telle qu'inférieure à 0,3 mm.

19. Un dispositif de délivrance de médication selon l'une des revendications précédentes, dans lequel une course de déplacement et/ou une course de restauration d'un cycle de pompage est/sont au moins partiellement actionnées par l'utilisateur du dispositif de délivrance de médication.

20. Un dispositif de délivrance de médication selon l'une des revendications précédentes, dans lequel une course de déplacement et/ou une course de restauration d'un cycle de la pompe est/sont au moins partiellement actionnées par un mécanisme à ressort.

21. Un dispositif de délivrance de médication selon l'une des revendications précédentes, comprenant en outre une aiguille hypodermique.
